Europäisches Patentamt

European Patent Office

Office européen des brevets

(19)

(11) Numéro de publication: **0 025 021**
**A1**

## (12) DEMANDE DE BREVET EUROPEEN

(21) Numéro de dépôt: **80830062.8**

(22) Date de dépôt: **01.08.80**

(51) Int. Cl.³: **A 61 B 17/36**
**F 25 B 9/02**

(30) Priorité: **02.08.79 IT 2488079**

(43) Date de publication de la demande:
**11.03.81 Bulletin 81/10**

(84) Etats Contractants Désignés:
**DE FR**

(71) Demandeur: **M.C.S. MEDICAL CONSTRUCTIONS**
**SERVICE S.R.L.**
**16122 Genova**
**Piazza Corvetto 1(IT)**

(72) Inventeur: **De Paz, Mario**
**Corso Magenta 23**
**Genova(IT)**

(74) Mandataire: **Calvani, Domenico**
**UFFICIO BREVETTI ING. CALVANI, SALVI & VERONELLI**
**4, Piazza Duca d'Aosta**
**I-20124 Milan(IT)**

(54) **Dispositif perfectionné destiné à des traitements de cryochirurgie et ensemble échangeur à haut rendement associé à ce dispositif.**

(57) Appareil de cryomédecine comprenant une cryode (12) qui est relié à une partie par laquelle le dispositif peut être tenu empoigné, et qui est alimentée en gaz, par exemple et de préférence du protoxyde d'azote hypersec, ce dispositif comporte un échangeur de chaleur (32) en contre-courant, cet échangeur étant relié à la petite masse métallique de la cryode (12) de manière à obtenir promptement et à grand rendement l'abaissement de la température de celle-ci.

Fig.2

EP 0 025 021 A1

DESCRIPTION de l'invention qui a pour titre :

"DISPOSITIF PERFECTIONNE DESTINE A DES TRAITEMENTS DE CRYO-
CHIRURGIE, ET ENSEMBLE ECHANGEUR A HAUT RENDEMENT ASSOCIE

A CE DISPOSITIF", au nom de la société dite "M.C.S. Medical

Constructions Service S.r.l.", à Gênes (Italie).

~~Déposée le    Août 1979, sous le n°~~

\* \* \* \* \* \*

La présente invention concerne un cryogénérateur, c'est-
à-dire un appareil destiné à permettre des interventions cliniques du type dit de cryochirurgie, au cours desquelles l'opérateur utilise la mise en contact avec la partie de corps destinée à subir l'intervention, d'un élément conformé métallique, dénommé cryode, amenée à une très basse température de refroidissement, de l'ordre de -90°C.

On connaît déjà des appareils conçus pour ces applications et leur emploi est largement répandu. Pour refroidir la

cryode on met à profit le principe de Joule-Thompson qui est appliqué en provoquant la détente, en milieu libre, d'un gaz sous pression, en particulier du protoxyde d'azote

hypersec et parfois d'autres gaz disponibles sous pression à l'état liquide et susceptibles d'une expansion brutale,

tel qu'un Fréon. Le protoxyde d'azote est, en général, préféré du fait qu'il permet d'atteindre la basse température voulue qui est plus adéquate au traitement cryochirurgical.

Dans les appareils connus, on fait détendre le gaz dans un milieu où se trouve situé un appendice de la cryode, lequel se refroidit et soustrait de la chaleur à la cryode elle-même. Pour atteindre la basse température de la cryode, ces appareils connus demandent une consommation importante de gaz et pour obtenir une expansion suffisante on fait généralement usage du protoxyde d'azote à l'état liquide qu' projette directement contre la cryode. D'autres problèmes, liés à l'usage desdits appareils connus, se posent pour obtenir un chauffage rapide de la cryode et de l'ensemble formant la partie à empoigner de l'appareil, pour éviter des phénomènes d'adhérence aux parties en cours de traitement et pour obvier à d'autres effets secondaires indésirés.

Ainsi, la présente invention a pour objet un appareil, en particulier un cryogénérateur destiné aux applications spécifiées ci-dessus, qui est perfectionné de manière à être exempt des problèmes et inconvénients des appareils de ce type disponibles à présent sur le marché.

Plus particulièrement, l'invention a pour objet un cryogébérateur perfectionné dont le constituant actif proprement dit, ou cryode, est capable de se refroidir très rapidement, ainsi que d'être ramené rapidement à une température tolérable, dans le cas d'interruption du traitement.

L'invention a également pour objet un cryogénérateur perfectionné dans lequel la cryode est thermiquement associée à un échangeur de chaleur à très grand rendement, qui

- 3 -

permet d'obtenir l'abaissement désiré de la température, et de maintenir la cryode à la température de service (préférablement de l'ordre de -90°C comme déjà dit) par une consommation très faible de protoxyde d'azote sous pression, en utilisant ledit protoxyde à l'état gazeux et à une pression relativement faible, de l'ordre de 50 atmosphères, gaz qui est envoyé dans le cryogénérateur et qui existe dans celui-ci à une basse pression, en vue d'éviter tout danger dans le cas de fuites accidentelles à proximité de personnes, en particulier d'un patient.

A titre d'exemple, le cryogénérateur perfectionné selon l'invention a confirmé expérimentalement d'être capable de fonctionner en continu avec une consommation de protoxyde d'azote gazeux de l'ordre de 1200 g/h, alors que, comme on le sait, les cryogénérateurs du commerce entraînent une absorbtion de gaz non inférieure à 3 kg/h.

Selon une caractéristique importante de l'invention, le cryogénérateur est associé à un échangeur de chaleur (formant élément caractérisant l'invention) travaillant en contre-courant, à grand rendement, et dans lequel ladite cryode forme une chambre qui se trouve en communication thermique avec le constituant qui matérialise la partie plus ou moins fuselée et amincie destinée à être mise en contact avec les tissus sur lesquels on se propose d'intervenir.

- 4 -

D'une manière essentielle, ledit échangeur de chaleur . est caractérisé en ce qu'il comporte à son intérieur, défini par une cavité recevant la partie interne de la cryode, au moins un double passage le long duquel le gaz est assujetti à une séquence de détentes, sous conditions de large contact thermique avec des parties métalliques, de façon à obtenir une soustraction rapide de chaleur, à haut rendement, en particulier et de préférence comme cela est spécifié dans la suite.

Ladite séquence de détentes, ou d'expansions, a essentiellement lieu dans des passages de petite section, mais dont l'intérieur est au moins en partie d'un dessin géométrique autre que celui cylindrique, ce qui permet d'accroître l'échange thermique et d'assurer l'obtention des résistances voulues au passage, qui ont pour conséquence de favoriser l'expansion isoenthalpique du gaz.

Selon une caractéristique avantageuse de l'invention, ledit échangeur, qui est pratiquement contenu dans la partie à empoigner du cryogénérateur, ou partie par laquelle on tient l'appareil, est facilement démontable pour permettre de remplacer la cryode en vue d'adapter l'appareil aux différentes conditions de manipulation et de traitement cryochirurgical, et aussi pour rendre aisé l'enlèvement des impuretés éventuelles, susceptibles d'être retenues dans les passages.

Selon une autre caractéristique avantageuse de l'invention, le corps creux de l'échangeur est associé à un moyen élastique susceptible de céder ou se relâcher dans le cas de production d'une surpression, en vue de permettre la décharge instantanée de cette surpression.

Ces caractéristiques et d'autres péculiarités de l'invention ressortent d'ailleurs de la description suivante d'un de ses modes de mise en oeuvre représenté, à titre d'exemple non limitatif, sur les dessins annexés, dans lesquel-:

- La figure 1 est une vue de côté, partie en coupe, de l'appareil dans son ensemble;

- La figure 2 est une vue en coupe et à plus grande échelle de l'extrémité recevant la cryode;

- La figure 3 est une vue fragmentaire, à plus grande échelle, des constituants extérieurs de l'appareil;

- La figure 4 est une vue éclatée des constituants intérieurs de l'échangeur;

- La figure 5 est une vue en coupe, à très grande échelle, du dispositif assemblé, la vue étant prise suivant le plan V-V de figure 1; et

- La figure 6 est une vue analogue à celle de figure 5 mais prise suivant VI-VI de figure 1.

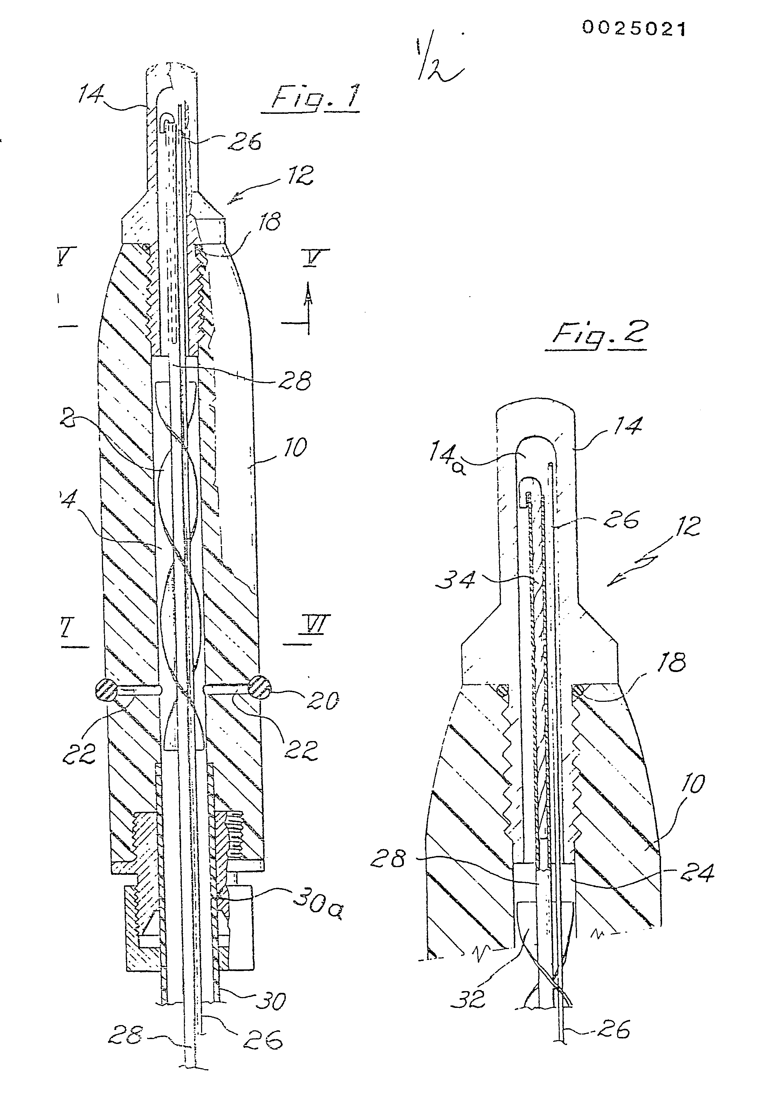

En ce référant aux figures ci-dessus et d'abord à la figure 1, qui montre dans son ensemble la structure du cryogénérateur perfectionné selon l'invention, on voit que cet appareil se compose d'une partie principale creuse 10, réalisée d'une manière avantageuse à partir d'un matériau à bas coefficient de conduction de la chaleur, par exemple un matériau à base de résines synthétiques, tel que le tétrafluoréthylène, ladite partie 10 étant associée à la cryode proprement dite —désignée par 12 dans son ensemble— qui est constituée par un élément en métal bon conducteur de la chaleur, tel que l'aluminium ou le cuivre, et qui est convenablement protégée, par chromage par exemple, en considération du fait que ce constituant est destiné à venir au contact du tissu devant être assujetti au traitement cryochirurgical. L'extrémité 14, opportunément fuselée, amincie ou conformée de toute autre façon appropriée, est la partie de l'appareil qui doit être mise en contact avec les tissus à traiter et qui doit, en conséquence, être refroidie et maintenue à la basse température désirée.

La cryode 12 est associée au corps 10, qui constitue la partie à empoigner de l'appareil, d'une façon facile à détacher et remplacer, par exemple par l'engagement vissé en 16a, 16b desdites pièces, et l'interposition d'une bague élastique —tel qu'un O-ring 18— assurant l'étanchéité.

En un point du corps à empoigner 10, considéré dans sa longueur, ce corps comporte une bague élastique ou anneau ressort 20 (figure 1) logé dans une gorge périmétrale qui

qui est en communication, à travers des passages 22, avec la cavité 24 intérieure audit corps. Grâce à cette disposition, dans le cas où une surpression devrait se produire dans la cavité 24, ladite bague élastique est capable de s'expanser ou s'élargir pour constituer ainsi un moyen de sécurité.

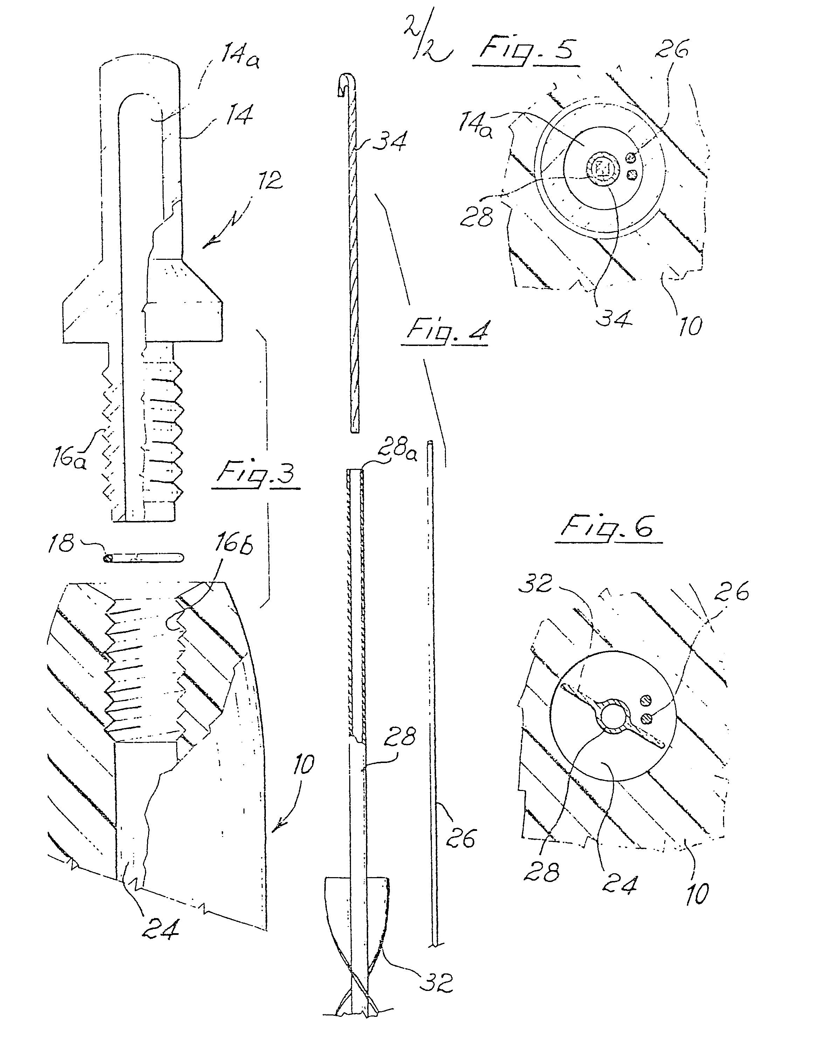

Les parties caractéristiques proprement dites de l'échangeur, visibles sur figures 1, sont représentées dans leurs différents détails aux figures 2-6.

Le cryogénérateur est relié -par l'entremise d'une soupape d'arrêt appropriée (séparée du cryogénérateur), placée dans un constituant statique qui comprend aussi un galvanomètre et un circuit amplificateur, ainsi que les piles alimentant le circuit d'amplification du signal d'un thermocouple, désigné schématiquement par 26 (figures 1,4-6)- à une source (une bouteille, en pratique) de protoxyde de azote gazeux hypersec, à travers un conduit 28 de diamètre intérieur relativement petit, par exemple de l'ordre de 1-2 mm. Ce conduit 28 est constitué par un tube métallique flexible qui est assez souple pour coopérer en flexion avec un tube extérieur 30 en matière plastique, tube qui forme le conduit de retour du gaz expansé et qui est relié en 30a à l'extrémité arrière de la partie à empoigner 10.

Le tube métallique 28, à sa partie intérieure à la cavité du cryogénérateur, est garni d'ailettes externes par exemple les ailettes hélicoïdales 32 (figures 1, 2 et 6) qui définissent une ample surface d'échange thermique. En outre, la partie d'extrémité intérieure dudit tube 28 reçoit une fine tige amovible 34 qui est avantageusement de section non circulaire et qui est façonnée ou torsadée de manière à augmenter d'elle-même la surface d'échange thermique et à provoquer des variations dans la section interne dudit tube 28. Ainsi, la partie terminale du tube 28, qui reçoit la tige amovible 34, forme un passage capillaire de section transversale discontinue, dans lequel il s'établit des conditions qui obligent le gaz à subir une expansion progressive, ce qui a pour conséquence un refroidissement. Le gaz sortant de l'extrémité 28a du tube 28 se décharge dans la cavité 14a de la cryode pour être ramené le long de la cavité 24 de la partie à empoigner 10, où le gaz se détend davantage en absorbant de la chaleur qui est enlevée aux ailettes 32.

Le cryogénérateur perfectionné incorpore, par conséquent, un échangeur de chaleur à deux étages, dans lequel il se produit absorption de chaleur, cette chaleur étant soustraite à l'unique constituant métallique existant, à savoir la cryode 12, laquelle se refroidit jusqu'à la température désirée. Cet échangeur de chaleur a montré d'avoir un haut rendement et d'être capable d'opérer à haute vitesse de réponse thermique, c'est-à-dire de pouvoir promptement et rapidement abaisser la température de la cryode, par exemple dans un laps de temps de l'ordre de 5s environ après l'ouverture de la soupape d'arrêt. De plus, puisque seul constituant métallique thermoconducteur de l'appareil est la cryode 12, laquelle est creuse et est convenablement configurée pour que sa masse thermique en soit réduite au minimum, cette cryode est à son tour capable de se chauffer très rapidement pour atteindre une température appropriée, non dangereuse en vu du contact avec les tissus à traiter, par exemple dans un laps de temps de l'ordre de 10-20 secondes depuis l'instant de la fermeture de ladite soupape d'arrêt, ce qui évite la nécessité de procéder à un chauffage de la pointe ou cryode, comme cela est généralement le cas, par contre, avec les appareils antérieurement connus.

Le fait d'avoir placé le thermocouple 26 tout près de la paroi interne de la cryode 12 permet d'assurer que l'élément sensible dudit thermocouple soit en état de mesurer effectivement la température de la cryode, c'est-à-dire d'en mesurer la température qui est réellement susceptible d'intérêt et qui est maintenue uniformément en tout point de la surface externe de ladite cryode.

Il va de soi que l'appareil formant l'objet de l'invention a été décrit ci-dessus et représenté sur les dessins annexés à titre d'exemple purement indicatif nullement limitatif de la portée de l'invention et que de nombreuses modifications et variantes pourront être apportées aux dif-

- 10 -

férents constituants de cet appareil, en vue d'adapter celui-ci à des exigences spécifiques de fabrication et d'application, sans sortir, pour autant, du cadre de l'invention, tel en particulier que défini dans l'une ou plusieurs
des revendications suivantes.

REVENDICATIONS

1. Dispositif perfectionné destiné à des traitements de cryochirurgie (cryogénérateur) comprenant une cryode qui est reliée à une partie par laquelle le dispositif peut être tenu ou empoigné, et qui est alimentée en gaz, par exemple et de préférence du protoxyde d'azote hypersec, caractérisé en ce que ce dispositif comporte un échangeur de chaleur en contre-courant, cet échangeur étant relié à la petite masse métallique de la cryode de manière à obtenir promptement et à grand rendement l'abaissement de la température de celle-ci.

2. Cryogénérateur perfectionné selon la revendication 1, caractérisé en ce que ce cryogénérateur incorpore un échangeur de chaleur qui comporte une séquence de passages à travers lesquels il se produit la détente en séquence du gaz qui permet d'obtenir le refroidissement voulu selon l'effet Joule-Thompson.

3. Cryogénérateur perfectionné selon les revendications 1 et/ou 2, caractérisé en ce qu'il comporte une partie à empoigner creuse, à l'intérieur de laquelle se trouvent situés les passages et les moyens d'échange thermique qui matérialisant le système opérant en contre-courant.

4. Cryogénérateur perfectionné selon la revendication 3, caractérisé en ce que ladite partie pouvant s'empoigner est formée d'un corps à basse conductibilité thermique, tel qu'un corps creux en un matériau à base de résines.

5. Cryogénérateur perfectionné selon une ou plusieurs des revendications 1 à 4, caractérisé en ce qu'il comprend une cryode métallique, en particulier en métal ou alliage métallique, ayant une haute conductibilité et un petite masse thermique.

6. Cryogénérateur perfectionné selon les revendications 4 et 5, caractérisé en ce que ladite cryode métallique est reliée avec ladite partie à empoigner de façon à pouvoir la séparer premptement de celle-ci.

7. Cryogénérateur perfectionné selon une ou plusieurs des revendications 1 à 6, caractérisé en ce que ledit échangeur de chaleur est réalisé à l'intérieur d'une cavité que délimitent entre elles la partie à empoigner et la cryode, ledit échangeur de chaleur étant en communication principalement thermique avec ladite cryode.

8. Cryogénérateur perfectionné selon la revendication 2 et, s'il y a lieu, l'une quelconque des revendications 1-7, caractérisé en ce que ledit échangeur de chaleur est matérialisé par la partie terminale d'un conduit métallique flexible de petite section, dans laquelle partie le gaz subit une première détente, et l'espace intérieur où se situent ladite partie terminale et la cavité qui est formée conjointement par la cryode et la partie à empoigner, et qui est parcourue en contre-courant par le gaz.

9. Cryogénérateur selon la revendication 8, caractérisé en ce que, dans ledit espace intérieur, ladite partie terminale du conduit métallique est garnie d'ailettes favorisant l'échange thermique.

10. Cryogénérateur perfectionné selon les revendications 8 et 9, caractérisé en ce qu'à l'intérieur de ladite partie terminale du conduit métallique se trouve placée une âme en forme de tige dont la présence permet de délimiter dans ladite partie terminale un passage sensiblement capillaire le long duquel le gaz est assujetti à une expansion progressive.

11. Cryogénérateur perfectionné selon la revendication 10, caractérisé en ce que le passage capillaire défini par ladite âme intérieure a une section discontinue sur sa longueur.

12. Cryogénérateur perfectionné selon la revendication 11, caractérisé en ce que ladite âme est formée d'une tige métallique de section non circulaire, torsadée.

13. Cryogénérateur perfectionné selon une ou plusieurs des revendications 1 à 12, caractérisé en ce qu'il comporte un système de sécurité susceptible de céder élastiquement dans le cas d'une surpression, ce système étant associé à la chambre dans laquelle l'échangeur de chaleur est formé.

14. Cryogénérateur perfectionné selon la revendication 13, caractérisé en ce que la partie à empoigner est pourvue d'une gorge extérieure qui communique avec ladite cavité à travers un ou plusieurs passages radiaux, ladite gorge recevant un anneau ressort susceptible de céder élastiquement et de s'expanser ou s'élargir localement dans le cas où une surpression devrait se manifester dans lesdits passages.

15. Cryogénérateur perfectionné selon la revendication 8 et une ou plusieurs des revendications 1 à 14, caractérisé en ce que ledit conduit métallique flexible est placé à l'intérieur d'un tube en matériau à base de résines qui forme -dans l'espace intermédiaire délimité entre ce tube et le conduit métallique- le passage de retour et de déchargement pour le gaz expansé.

16. Cryogénérateur perfectionné selon une ou plusieurs des revendications 1 à 13, caractérisé en ce que ce cryogénérateur comporte un thermocouple sensible à la température de la cryode, ce thermocouple étant relié, par l'entremise d'un circuit amplificateur, à un instrument indiquant la température instantanée de la cryode.

17. Cryogénérateur perfectionné selon la revendication 16, caractérisé en ce que la partie sensible dudit thermocouple se trouve placée à l'intérieur de la cryode à un point tout près des parois intérieures de celle-ci, de telle façon que l'indication ainsi obtenue corresponde effectivement à celle fournie par ladite cryode.

*Fig.1*

*Fig.2*

0025021

2/2  _Fig. 5_

_Fig. 4_

_Fig. 3_

_Fig. 6_

**Office européen des brevets**

## RAPPORT DE RECHERCHE EUROPEENNE

Numéro de la demande

EP 80 83 0062

| | DOCUMENTS CONSIDERES COMME PERTINENTS | |
|---|---|---|
| Catégorie | Citation du document avec indication, en cas de besoin, des parties pertinentes | Revendica-tion concernée |
| X | <u>FR - A - 2 207 730</u> (DRAGERWERK AKTIENGESELLSCHAFT)<br><br>* Revendications; figures * | 1-4,7, 8,15-17 |
| | -- | |
| X | <u>US - A - 3 951 152</u> (CRANDELL)<br><br>* Colonne 4, ligne 11 - colonne 6, ligne 16; figures * | 1-5,7-9,15-17 |
| | -- | |
| | <u>FR - A - 2 063 828</u> (HUGHES AIR-CRAFT COMPANY)<br><br>* Figures; page 3, lignes 6-27 * | 1-3,7, 9 |
| | -- | |
| | ENGINEERING, vol. 205, juin 1968, page 985<br>London, G.B.<br>"Cryogenic probe for surgery"<br><br>* Page 985 en entier * | 1,4-6 |
| | ---- | |

**CLASSEMENT DE LA DEMANDE (Int. Cl. 3)**

A 61 B 17/36
F 25 B 9/02

**DOMAINES TECHNIQUES RECHERCHES (Int. Cl. 3)**

A 61 B
F 25 B
F 25 J
F 16 K

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 04-11-1980 | STEENBAKKER |

OEB Form 1503.1   06.78